(19) Europäisches Patentamt
European Patent Office
Office européen des brevets

(11) Numéro de publication: **0 100 745**
**B1**

# (12) FASCICULE DE BREVET EUROPEEN

(45) Date de publication du fascicule du brevet: **26.02.86**

(51) Int. Cl.⁴: **C 07 D 307/90**, A 61 K 31/495

(21) Numéro de dépôt: **83450013.4**

(22) Date de dépôt: **19.05.83**

(54) **Nouvelles ((phényl-4 pipérazinyléthyl)-2 aniline)-3 isobenzofurannones, leur méthode de préparation et leur application thérapeutique.**

(30) Priorité: **29.07.82 FR 8213446**

(43) Date de publication de la demande:
**15.02.84 Bulletin 84/7**

(45) Mention de la délivrance du brevet:
**26.02.86 Bulletin 86/9**

(84) Etats contractants désignés:
**BE CH DE GB IT LI LU NL**

(56) Documents cités:
**CH - A - 398 614**
**FR - A - 2 168 931**
**FR - E - 85 503**

(73) Titulaire: **SOCIETE CORTIAL S.A., 7 rue de l'Armorique, F-75015 Paris (FR)**

(72) Inventeur: **Creuzet, Marie-Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Guichard, Françoise, Les Cèdres-Parc des Tourelles Rue St. Elisabeth, F-33200 Bordeaux (FR)**
Inventeur: **Prat, Gisèle, Hameau de Noailles Villa 18, F-33400 Talence (FR)**
Inventeur: **Pontagnier, Henri, 21 rue Edouard Vaillant, F-33600 Pessac (FR)**

(74) Mandataire: **Tajan, Marie-Thérèse, LABORATOIRES SARGET Avenue du Président JF Kennedy, F-33701 Mérignac (FR)**

**EP 0 100 745 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention concerne de nouvelles benzoisofurannones, leur méthode de préparation ainsi que leur emploi en thérapeutique.

Ces dérivés ont pour formule générale

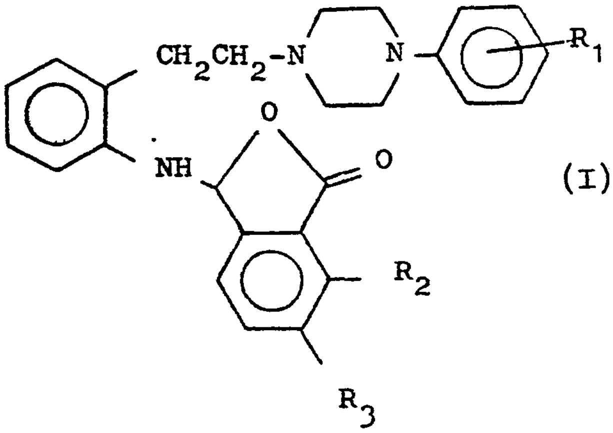

(I)

avec $R_1$ = H ou un ou plusieurs substituants situés en ortho, méta ou para choisi parmi $CH_3$, $CF_3$, F, Cl, $OCH_3$; $R_2$, $R_3$: identiques ou différents = H ou $OCH_3$. Ces produits peuvent être utilisés sous forme de base libre ou de sels pharmaceutiquement compatibles tels que les chlorhydrates, citrates, benzilates.

On connaissait déjà des imines de structure

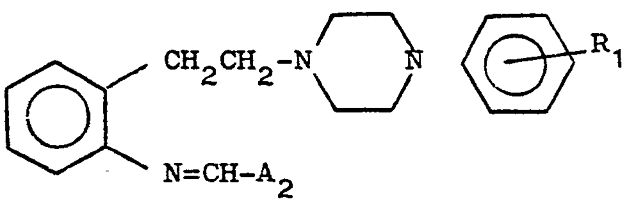

décrites dans le brevet français numero 2.515.647 du 3 novembre 1981. Les produits selon la présente invention du fait de l'existence d'un fragment -O-CH-NH- remplaçant la liaison -CH=N- présente dans les imines possedent des caractéristiques physicochimiques différentes. Ainsi leur stabilité en milieu acide est plus importante que celle des imines correspondantes. Cela se traduit sur le plan de l'application thérapeutique par des différences notables. des propriétés antianaphylactiques, antihistaminiques, antisérotoninergique s, antihypertensives permettant leur emploi en thérapeutique par exemple en allergologie ou en thérapeutique cardiovasculaire. une réaction de condensation entre des amines de formule générale

(II)

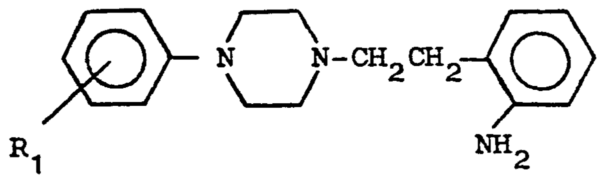

avec $R_1$ = H ou un ou plusieurs substituants situés en ortho, méta ou para choisis parmi $CH_3$, $CF_3$, F, Cl, $OCH_3$, amines décrites dans le brevet français numéro 2.515.648 du 3.11.1981 et un aldéhyde aromatique comportant en ortho de la fonction carboxaldéhyde une fonction acide

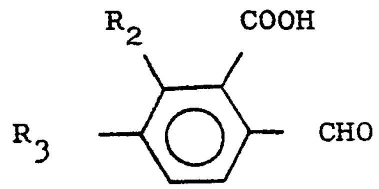

(III)

avec $R_2$, $R_3$ identiques ou différents = H ou $OCH_3$.

On chauffe le mélange des produits de départ en prisence d'un solvant choisi parmi le benzène, l'eau étant éliminée au fur et à mesure de la réaction.

La présente invention va être décrite de façon plus précise dans les exemples suivants.

### Exemple 1

Synthèse de la ((phényl-4 pipérazinyléthyl)-2 anilino)-3 isobenzofurannone; produit de formule I avec $R_1 = R_2 = R_3 =$ H; nom de code COR 34 30

On chauffe au reflux du benzène sous agitation, le mélange constitué de 0,025 mole de (phényl-4 pipérazinyléthyl)-2 aniline, 0,023 mole d'acide au fur et à mesure de sa formation à l'aide d'un piège de Dean et Stark; lorsque la réaction est complète, le benzène est évaporé; le résidu d'évaporation est cristallisé dans un mélange d'éther éthylique et d'éther de pétrole. Le solide est filtré et recristallisé dans l'éthanol. On obtient ainsi le produit de l'exemple 1 avec un rendement de 77 %. Point de fusion 180°C. Masse moléculaire 413,5. Microsnalyse: calculé C 75,52 H 6,57 N 10,16 0 7,74,; trouvé C 75,76 H 6,66 N 10,03 H 7,54; RMN dans $CDCl_3$ 2,3-3,0 ppm, 12 protons, massif complexe ($CH_2$); 6,4-7,7 ppm, 15 protons, massif complexe (protons aromatiques + NH-CH-).

**Exemple 2**

Synthèse de la ((p-méthoxyphényl-4 pipérazinyléthyl)-2 anilino)-3 isobenzofurannone; produit de formule I avec $R_1 = OCH_3$-4, $R_2 = R_3$ = H; nom de code COR 34 53.

Ce produit est préparé selon la méthode décrite dans l'exemple 1 à partir de paraméthoxyphényl-4 pipérazinyléthyl-2 aniline et d'acide phtalaldéhydique 73,11 H 6,59 N 9,47 O 10,82; trouvé C 72,53 H 6,61 N 9,42 O 11,51. RMN dans $CDCl_3$ 2,1-3,0 ppm, 12 protons, massif complexe (CH); 3,7 ppm, 3 protons, singulet ($OCH_3$); 6,4-7,8 ppm, 14 protons, massif complexe (protons aromatiques + -NH-CH-)

**Exemple 3**

Synthèse de la ((p-fluorophényl-4 pipérazinyléthyl)-2 anilino)-3 isobenzofurannone; produit de formule I avec $R_1$= F-4,$R_2$= $R_3$= H; nom de code COR 34 37.

Ce produit est préparé selon la méthode décrite dans l'exemple 1 à partir de p-fluorophénylpipérazinyl éthyl-2 aniline et d'acide phtalaldéhydique svec un rendement de 84 %. Masse moléculaire 431,5. Point de fusion 184°C. Microanalyse: calculé C 72,37 H 6,06 N 9,74 F 4,40; trouvé C 72,09

H 6,35 N 9,51 F 4,41. RMN dans $CDCl_3$ 2,2-3,0 ppm, 12 protons, massif complexe ($CH_2$); 6,4-7,8 ppm, 14 protons, massif complexe (protons aromatiques + -NH-CH-)

**Exemple 4**

Synthèse de la (((méthoxy-2 phényl)-4 pipérazinyléthyl)-2 anilino)-3 isobenzofurannone; produit de formule I avec $R_1 = OCH_3$-2, $R_2 = R_3$ = H; nom de code COR 34 41.

Ce produit est préparé selon la méthode décrite dans l'exemple 1 à partir de (méthoxy-2 phényl)-4 pipérazinyléthyl-2 aniline et d'acide phtalaldéhydique avec un rendement de 82 %. Masse moléculaire 443,5. Point de fusion 173°C. Microanalyse: calculé C 73,11 H 6,59 N 9,47 0 10,82; trouvé C 72,75

H 6,58 N 9,42 0 10,59. RMN dans $CDCl_3$ 2,2-3, Ippm, 12 protons, massif complexe ($CH_2$); 3,7 ppm, 3 protons, singulet ($CH_30$); 6,3-8,0 ppm, 14 protons, massif complexe (protons aromatiques + -NH-CH-)

**Exemple 5**

Synthèse de la (((methyl-2 phenyl)-4 pipérazinyléthyl)-2 anilino)-3 isobenzofurannone; produit de formule I avec $R_1 = CH_3$-2, $R_2 = R_3$ = H; nom de code COR 34 49.

Ce produit est préparé selon la méthode décrite dans l'exemple 1 à partir de (méthyl-2 phényl)-4 pipérazinyléthyl-2 aniline et d'acide phtalaldéhydique avec un rendement de 67 %. Masse moléculaire 427,6. Point de fusion 174°C. Microanalyse: calculé C 75,85 H 6,84 N 9,83 0 7,4ô; trouvé C 75,61 H

6,76 N 9,91 0 7,49. RMN dans $CDCl_3$ 2,0-3,0 ppm, 15 protons, massif complexe ($CH_2 + CH_3$); 6,3-8,0 ppm, 14 protons, massif complexe (protons aromatiques + -NH-CH-).

**Exemple 6**

Synthèse de la diméthoxy-6,7 ((phényl-4 pipérazinyléthyl)-2 anilino)-3 isobenzofurannone; produit,de formule I avec $R_1$ = H, $R_2 = R_3$ = $CH_30$; nom de code COR 34 47.

Ce produit est préparé selon la méthode décrite dans l'exemple 1 à psrtir de phényl-4 pipérazinyléthyl-2 aniline et d'acide opianique avec un rendement de 78 %. Masse moléculaire 473,6. Point de fusion 199°C. Micruanalyse: calculé C 71,01 H 6,60 N 8,87 0 13,51; trouvé C 70,59 H 6,60 N 8,75 0 13,84. RMN dans $DMSOD_6$ 2,4-3,3 ppm, 12 protons, massif complexe ($CH_2$); 3,6 et 3,8 ppm, 6 protons, singulets (2 $OCH_3$); 6,6-8,1 ppm, 13 protons, massif complexe (protons aromatiques + -NH-CH-)

**Exemple 7**

Synthèse de la dimethoxy-6,7 ((p-fluorophényl-4 pipérazinyléthyl)-2 anilino)-3 isobenzofurannone; produit de formule I avec $R_1$ = F-4, $R_2$ $R_3$ = $OCH_3$; nom de code COR 34 43.

Ce produit est préparé selon la méthode décrite dans l'exemple 1 à partir un rendcment de 82 %. Masse moléculaire 493,5. Point de fusion 210°C. Microanalyse: calculé C 68,42 H 6,15 N 8,54 F 3,86; trouvé C 68,44 H 6,08 N 8,48 F 3,77. RMN dans le $DMSOD_6$ 2,4-3,1 ppm, 12 protons, massif complexe ($CH_2$); 3,6 et 3,8 ppm, 6 protons, singulets (2 $OCH_3$); 6,5-7,7 ppm, 12 protons, massif complexe (protons aromatiques + -NH-CH-)

**Exemple 8**

Synthèse de la ((p-chlorophényl-4 pipérazinyléthyl)-2 anilino)-3 diméthoxy-6,7 isobenzofurannone; produit de formule I avec $R_1$ = Cl-4 $R_2$ = $R_3$ = $OCH_3$; nom de code COR 34 42.

Ce produit est preparé selon la méthode decrite dans l'exemple 1 à partir de p-chlorophenyl-4 pipérazinyléthyl-2 aniline et d'acide opianique avec un rendement de 58 %. Masse moléculaire 508,0. Point de fusion 199°C. Microanalyse: calculé C 66,20 H 5,95 N 8,27 0 12,60 Cl 6,98; trouvé C 66,08 H 5,98 N 7,53 O 13,27 Cl 6,79. RMN dans le DMSOD6 2,4-3,1 ppm, 12 protons, massif complexe ($CH_2$); 3,6 et 3,8 ppm, 6 protons, singulets (2 $OCH_3$); 5,6 ppm, 1 proton, dôme (NH); 6,6-7,5 ppm, 11 protons, massif complexe (protons aromatiques + $-\underset{|}{CH}-$)

**Exemple 9**

Synthèse de la diméthoxy-6,7 (((methyl-2 phényl)-4 piperazinyléthyl) -2 anilino)-3 isobenzofurannone; produit de formule I avec $R_1$ = $CH_3$-2 $R_2$ = $R_3$ = $OCH_3$; nom de code COR 34 48.

Ce produit est préparé selon la méthode décrite dans l'exemple 1 à partir de ((methyl-2 phényl)-4 pipérazinylethyl)-2 aniline et d'acide opianique avec un rendement de 88 %. Masse moléculaire 487,6. Point de fusion 180°C. Microanalyse: calculé C 71,43 H 6,82 N 8,62 0 13,12; trouvé C 71,42 H 6,86 N 8,69 0 13,27. RMN dans $DMSOD_6$ 2,2 ppm, 3 protons, singulet ($CH_3$-C); 2,4-3,2 ppm, 12 protons, massif complexe ($CH_2$); 3,7-3,8 ppm, 6 protons, singulet (2 $OCH_3$); 6,6-8,1 ppm, 12 protons, massif complexe (protons aromatiques + $-NH-\underset{|}{CH}-$).

Les produits faisant l'objet de la présente invention ont subi divers tests pharmacologiques dont nous donnons ccrtains résultats ci-après.

La mortalité induite par ces substances a été déterminée chez la souris Swiss exempte d'organisme pathogène spécifique. Les animaux sont stabulés en salle climatisée 24 à 48 heures avant le debut de l'experimentation. Les substances sont administrées soit par voie intragastrique soit par voie intrapéritonéale en solution dans du Twéen. Les resultats sont exprimés dans les tableaux I et II par le pourcentage de mortalite en fonction de la dose administréc ou par la dose induisant 50 % de mortalité (DL 50) determinée par la méthode de Bliss.

**TABLEAU I**

Toxicité par voie orale

| PRODUIT | Doses en mg/kg | | | |
|---|---|---|---|---|
| | 750 | 1000 | 2000 | 3000 |
| COR34 30 | | 0 | 0 | 10 |
| COR34 37 | 0 | 10 | 50 | |
| COR34 41 | 0 | 10 | 10 | |
| COR34 42 | | 0 | 0 | |
| COR34 43 | | 0 | 0 | |
| COR34 47 | | 0 | 0 | |
| COR34 48 | | 0 | 0 | |
| COR34 49 | | 0 | 0 | |
| COR34 53 | | 0 | 20 | |

**TABLEAU II**

Toxicité par voie intrapéritonéale

| PRODUIT | DOSE EN mg/kg | | | | | | | | DL 50 |
|---|---|---|---|---|---|---|---|---|---|
| | 100 | 200 | 300 | 400 | 500 | 800 | 900 | 1000 | en mg/kg |
| COR34 30 | 0 | 10 | 0 | | 0 | | | | |
| COR34 37 | | | | | 20 | | | 60 | |
| COR34 41 | | | | 0 | 25 | 35 | 50 | 70 | 873(700-1091) |
| COR34 43 | | | | | 0 | 0 | | 0 | |
| COR34 47 | | | | | 0 | | | 0 | |
| COR34 48 | | | | | 0 | | | 0 | |
| COR34 49 | | | | | 0 | | | 20 | |
| COR34 53 | | | | | 60 | | | 100 | 500(429-582) |

L'activite antihistaminique a été determinée in vitro par la mesure de le concentration entrainant 50 % d'inhibition des contractions induites par l'histamine ($10^{8}$ g/l) sur iléon de cobaye (Cl 50). Ces resultats sont représentes dans le tableau 3. L'antagoniste est administré en même temps que l'agoniste ou avant. Dans ce dernier cas est indiqué dans le tableau 3 le temps de contact entre l'antagoniste et l'organe isole avant l'introduction de l'agoniste.

## TABLEAU III

Activité antihistaminique in vitro

| PRODUIT | CI 50 en M/l | Temps de contact |
|---|---|---|
| COR34 30 | $1{,}04.10^{-6}(4{,}91.10^{-7}-2{,}20.10^{-6})$ | |
| COR34 37 | $1{,}55.10^{-8}(1{,}11.10^{-8}-2{,}17.10^{-8})$ | 3 mn |
| COR34 41 | $3{,}11.10^{-8}(2{,}55.10^{-8}-3{,}86.10^{-8})$ | 10 mn |
| COR34 42 | $3{,}3.10^{-8}(2{,}7.10^{-8}-4{,}01.10^{-8})$ | 5 mn |
| COR34 43 | $3{,}53.10^{-6}(2{,}76.10^{-6}-4{,}54.10^{-6})$ | |
| COR34 47 | $2{,}9.10^{-8}(1{,}9.10^{-8}-4{,}6.10^{-8})$ | 3 mn |
| COR34 48 | $5{,}5.10^{-8}(3{,}34.10^{-8}-9{,}04.10^{-8})$ | 5 mn |
| COR34 49 | $8.10^{-8}(6{,}3.10^{-8}-1.10^{-7})$ | 10 mn |
| COR34 53 | environ $1{,}5.10^{-6}$ | 5 mn |
| Prométhazine | $4{,}93.10^{-9}(3{,}10.10^{-9}-7{,}83.10^{-9})$ | 15 mn |
| Chlorpromazine | $3{,}63.10^{-6}(2{,}18.10^{-6}-4{,}20.10^{-6})$ | |

Les produits de la présente invention sont des entagonistes compétitifs de l'histamine; le COR34 30 présente un $pA_2$ de 7 69, le COR34 37 un pA de 7,86, le COR34 41 un $pA_2$ de 8,03 et le COR 34 43 un $pA_2$ de 7,97.

Le tableau n°4 indique l'activité antisérotonine des produits de la présente invention. Cette activité est exprimée par la concentration de substance inhibant in vitro 50 % des contractions induites par le sérotonine (5 10-7 g/ml) sur un iléon isolé de cobaye (CI 50)

## TABLEAU IV

Activité antisérotonine

| PRODUIT | CI 50 en M/l | Temps de contact |
|---|---|---|
| COR34 30 | $9{,}33.10^{-6}(5{,}39.10^{-6}-1{,}62.10^{-5})$ | 5 mn |
| COR34 37 | $2{,}27.10^{-5}(2{,}16.10^{-5}-2{,}39.10^{-5})$ | 5 mn |
| COR34 41 | $1{,}26.10^{-5}(9{,}45.10^{-6}-1{,}70.10^{-5})$ | 15 mn |
| COR34 43 | $2{,}05.10^{-5}(1{,}45.10^{-5}-2{,}90.10^{-5})$ | 3 mn |
| COR34 47 | $1{,}97.10^{-5}(1{,}66.10^{-5}-2{,}38.10^{-5})$ | 3 mn |
| COR34 48 | $6{,}46.10^{-6}(5{,}04.10^{-6}-8{,}31.10^{-6})$ | 5 mn |
| Prométhazine | $4{,}44.10^{-7}(2{,}69.10^{-7}-7{,}32.10^{-7})$ | 3 mn |
| Cyproheptadine | $1{,}41.10^{-7}(8{,}56.10^{-8}-2{,}34.10^{-7})$ | 3 mn |

In vivo l'activité antihistaminique est déterminée chez le cobaye éveillé dans le test du bronchospasme induit par un aérosol d'histamine à 0,3 %. Les produits sont administrés par voie orale en solution dans du Twéen.

Sont donnés dans le tableau n° 5 les pouréentages d'animaux protégés au bout de 1 h et 6 h à la dose de 10 mg/kg.

## TABLEAU V

Activité antihistaminique in vivo

| PRODUIT | % d'animaux protégés à | |
|---|---|---|
| | 1 h | 6 h |
| COR34 30 | 60 | 40 |
| COR34 37 | 70 | 70 |
| COR34 41 | 90 | 60 |
| COR34 43 | 90 | 50 |
| COR43 47 | 20 | 0 |
| COR34 48 | 40 | 10 |
| COR34 49 | 30 | 20 |

L'activité antiallergique a été déterminée dans le test de l'anaphylaxie passive cutanée chez le rat Sprague Dawley. L'anaphylaxie est induite par l'administration intradermique d'un sérum riche en IgE puis 24 heures après par l'injection intraveineuse d'une solution contenant de l'ovalbumine et du bleu d'Evans. L'activité des produits à tester administrés per os est appréciée par le pourcentage de diminution de la quantité du bleu d'Evans ayant diffusé au point d'injection. Le tableau n°6 donne exprimées en mg/kg les DE 50 de certains produits de la présente invention.

Les produits de la présente invention ont été testés par voie orale dans le modèle des réactions cutanées induites par administration intradermique d'histamine, de sérotonine ou du composé 48/80 chez le rat.

L'activité des produits est appréciée par le pourcentage de diminution de la quantité de bleu ayant diffusé au point d'injüction et les DE 50 exprimées en mg/kg sont données dans le tableau 6.

**TABLEAU VI**

| PRODUIT | ANAPHYLAXIE passive cutanée | REACTIONS CUTANEES INDUITES PAR histamine | sérotonine | 48/80 |
|---|---|---|---|---|
| COR34 30 | 3,8(2,5-5,7) | 12,1(8,5-17) | 13,2(8,7-19,9) | 11,2(7,9-15,9) |
| COR34 37 | 6,4(4,9-8,5) | 9,3(6.6-13.2) | 7,9(6,0-10,5) | 9,4(7,1-12,3) |
| COR34 61 | 4,1(2,3-7,1) | 7,9(5,3-12,0) | 14,0(8,7-22,9) | 10,9(6,3-19,0) |
| COR34 62 | 14,8(9,1-24) | | | |
| COR34 63 | 6,6(4,1-10,7) | 9,1(4,9-17) | 17,1 (13,8-20,9) | 11,1(7,2-16,6) |

Les produits de la présente invention présentent une activité antihypertensive dont on donne ci-aprés quelques exemples.

Ainsi chez le rat spontanément hypertendu vigile le COR 34 41 entraine à 25 mg/kg per os une chute de pA de 18,8 % et à 50 mg/kg, une chute de pA de 19,3 %. La pA revient à la normale au bout d'une heure à la dose de 25 mg/kg et au bout de 5 heures à la dose de 50 mg/kg. Le COR34 42 à la dose de 50 mg/kg entraine une chute de pA de 15,6 %; la pA revient à la normale au bout de 5 heures. Administré pendant 5 jours par voie orale à des rats spontanémentt hypertendus à raison d'une dose de 50 mg/kg toutes les 12. heures le COR 34 41 entraine une chute de la PA, celle-ci se stabilisant à environ 18 mm Hg au dessous de sa valeur initiale en fin de traitement. chez la souris en utilisant un actimètre. Les DE 50 sédatives sont de 50 mg/kg pour le COR34 30 10 mg/kg pour le 34 42, 18 mg/kg pour le 34 49, 9 mg/kg pour le COR34 48.

Compte tenu de leurs propriétés antihistaminiques, antisérotoninergiques et antianaphylactiques jointes à une faible toxicité, les produits de la présente invention sont utiles en thérapeutique employés seuls ou en association dans le traitement des états allergiques et anaphylactiques tels que l'urticaire, les prurits, les dermatoses, les eczémas, le rhume des foins, l'oedème de Quincke, la maladie sérique, l'asthme, le choc anaphylactique. Ils peuvent être employés à titre préventif ou curatif dans le traitement du mal des transports. Les propriétés antihypertensives permettent de les utiliser seul ou en association avec un diurétique et/ou d'autres médicaments antihypertenseuis dans le traitement de l'hypertension artérielle.Ils seront administrés par exemple par voie orale sous forme de dragées, comprimés, sirop, ampoules, par voie rectale sous forme de suppositoires, par voie intramusculaire ou intraveineuse ou par voie topique sous forme de pommades ou gels. Les doses administrées varieront selon l'indication et le sujet de 1 à 100 mg/kg en 2 à 6 prises pour la voie orale, de 1 à 100 mg/j en 1 ou 2 prises pour la voie rectale, de 0,5 à 50 mg par injection pour les voies parentérales.

**Revendications**

1 - produits de formule générale

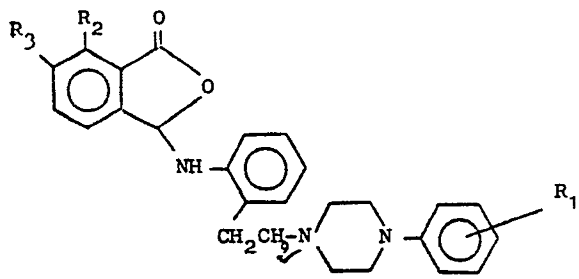

ainsi que leurs sels d'addition non toxiques, formule dans laquelle $R_1$ = H ou un ou plusiuers substiuants situés en ortho, méta ou choisis parmi $CH_3$, $CF_3$., F, Cl, $OCH_3$; $R_2$, $R_3$ identiques ou différents = H ou $OCH_3$.

2 - procédé de préparation des produits selon la revendication 1 formule

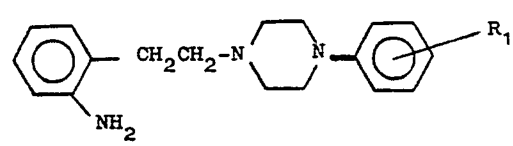

avec $R_1$ = H ou un ou plusieurs substituants situés en ortho, méta ou para choisis parmi $CH_3$, $CF_3$, F, Cl, $OCH_3$, d'un aldéhyde de formule

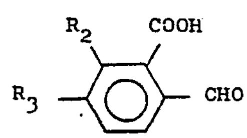

avec $R_2$, $R_3$ identiques ou différents = H ou $OCH_3$ et d'un solvant choisi parmi le benzène, l'eau étant éliminée au fur et à mesure de la réaction.

3 - Medicament caractérisé en ce que le principe actif est constitué par au moins un produit selon la revendication 1.

4 - Composition pharmaceutique ou vétérinaire caractérisée en ce qu'elle en association avec un véhicule pharmaceutique ou un excipient approprié.

## Claims

1. Products with the general formula

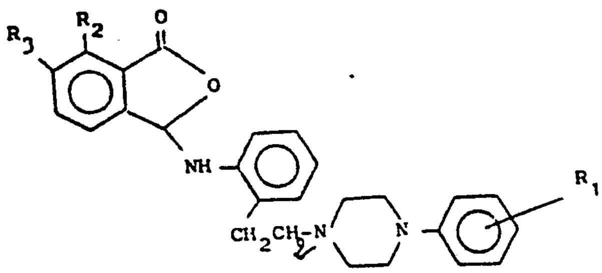

as well as the non-toxic addition salts thereof, formula wherein $R_1$ = H or one or a plurality of ortho-, meta- or para-located substituents, selected among $CH_3$, $CF_3$, F, C1, $OCH_3$; $R_2$, $R_3$, identical or different, = H or $OCH_3$.

2. Method for preparing products as defined in claim 1, which comprises heating the mixture comprised of an amine with as formula

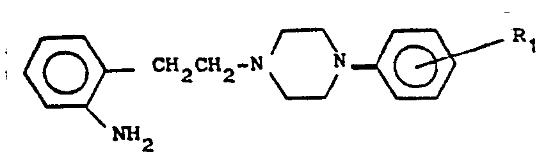

in which $R_1$ = H or one or a plurality of ortho-, meta- or paralocated substituents selected among $CH_3$, $CF_3$, F, Cl, $OCH_3$, an aldehyde with as formula

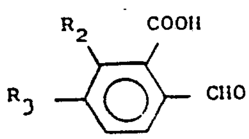

in which $R_2$, $R_3$, identical or different, = H or $OCH_3$, and a solvent selected among the benzene, the water being removed as the reaction proceeds.

3. Medicament, in which the active constituent is comprised of at least one product as defined in claim 1.

4. Pharmaceutic or veterinary compound, which contains as active constituent at least one product as defined in claim 1 in association with a suitable pharmaceutic vehicle or excipient.

## Patentansprüche

1. Verbindungen der allgemeinen Formel

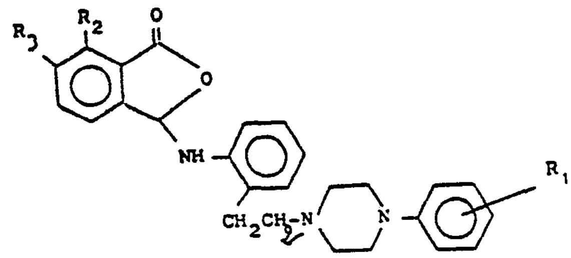

sowie ihre nicht toxischen Additionssalze, wobei in der Formel $R_1$ = H oder einen in ortho-, meta- oder para-Stellung bef indlichen Substituenten, ausgewählt aus der von $CH_3$, $CF_3$, F, Cl und $OCH_3$ gebildeten Gruppe, oder mehrere dieser Substituenten, und $R_2$ und $R_3$, die gleich oder verschieden sein können, H oder $OCH_3$ bedeuten.

2. Verfahren zur Herstellung der Verbindungen nach Anspruch 1, dadurch gekennzeichnet, daß man ein Gemisch aus einem Amin der Formel

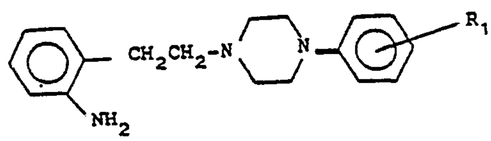

in der $R_1$ = H oder einen in ortho-, meta- oder para-Stellung befindlichen Substituenten, ausgewählt aus der von $CH_3$, $CF_3$, F, Cl und $OCH_3$ gebildeten Gruppe, einem Aldehyd der Formel

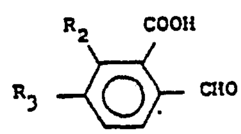

in der $R_2$ und $R_3$, die gleich oder verschieden sein können, H oder $OCH_3$ bedeuten, und einem Lösungsmittel wie Benzol erhitzt, wobei man das im Verlauf der Reaktion gebildete Wasser entfernt.

3. Medikament, dadurch gekennzeichnet, daß sein Wirkstof f aus mindestens einer Verbindung nach Anspruch 1 besteht.

4. Pharmazeutische oder veterinärmedizinische Zusammensetzung, dadurch gekennzeichnet, daß sie als Wirkstoff mindestens eine Verbindung nach Anspruch 1 zusammen mit einem pharmazeutischen Träger oder einem geeigneten Exzipienten enthält.